# EUROPÄISCHE PATENTSCHRIFT

(11) **EP 3 592 327 B1**
(45) Veröffentlichungstag und Bekanntmachung des Hinweises auf die Patenterteilung: **11.08.2021**
(21) Anmeldenummer: 18708110.4
(22) Anmeldetag: 28.02.2018
(51) Int. Cl.: A61K 8/34, A61Q 1/04, A61K 8/92, A61K 8/02

(54) **LIPIDMISCHUNG AUS OCTYLDODECANOL UND HYDRIERTEM RAPSÖL**
LIPID MIXTURE OF OCTYLDODECANOL AND HYDROGENATED RAPESEED OIL
MELANGE LIPIDIQUE D'OCTYLDODECANOL ET D'HUILE DE RAPE HYDRATÉE

(30) Priorität: 07.03.2017 DE 102017203641
(43) Veröffentlichungstag der Anmeldung: 15.01.2020
(73) Patentinhaber: Beiersdorf AG, 20253 Hamburg (DE)
(72) Erfinder: ZEWUHN, Merle, 25335 Elmshorn (DE); FIEDLER, Dorothe, 22335 Hamburg (DE); RESHAD, Sepideh, 22175 Hamburg (DE)
(86) Internationale Anmeldenummer: PCT/EP2018/054879
(87) Internationale Veröffentlichungsnummer: WO 2018/162288

(56) Entgegenhaltungen:
- WO-A1-99/63031
- WO-A1-2012/131624
- WO-A1-2017/008243
- DE-A1-102014 204 477
- DE-U1- 20 309 463
- DATABASE GNPD [Online] MINTEL; Juli 2013 (2013-07), "Lipstick", XP002780138, Database accession no. ID2119122

## Beschreibung

Die vorliegende Erfindung betrifft eine kosmetische Lipidmischung aus Octyldodecanol und hydriertem Rapsöl im Gewichtsverhältnis von 7:1 bis 1:1, ein Verfahren zur Herstellung der Lipidmischung sowie einen Lippenstift enthaltend diese Lipidmischung.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Hautpflegeprodukte, in der Regel Cremes, Salben oder Lotionen, dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Neben der Reinigung und Pflege der Haut haben Kosmetika auch eine ästhetische Aufgabe. Sie sollen das äußere Erscheinungsbild des Anwenders entsprechend den jeweiligen kulturellen Vorstellungen "verbessern". Kosmetika erfüllen damit eine psychologisch-soziale Funktion, da sie die (optische) Attraktivität der Anwender erhöhen. In diesen Bereich fällt vor allen Dingen die "dekorative" Kosmetik, die mit Hilfe von auf die Haut aufgetragenen Farbstoffen das Erscheinungsbild der Anwender verändert. Indirekt haben aber auch Reinigungs- und Pflegeprodukte einen positiven Einfluss, da eine saubere, gesunde Haut dem Schönheitsideal der Menschen entspricht.

Eine besondere Form kosmetischer Zubereitungen stellen die Lippenpflegeprodukte dar. Sie dienen einerseits der Pflege der Lippenhaut und schützen bzw. regenerieren die Lippen bei Trockenheit und Rissigkeit. Andererseits dienen Lippenpflegeprodukte auch zur Dekoration, da sie den Lippen durch Glanzeffekte oder Farbe zu einem besonderen Ausdruck verhelfen. Lippenpflegeprodukte werden sowohl in Stiftform oder als mehr oder weniger zähfließende Crememasse ("Lippenbutter") dargereicht.

An Lippenpflegeprodukte werden besondere Anforderungen gestellt. Sie müssen in toxikologischer Hinsicht vollkommen unbedenklich sein um jegliche Gefahr einer Vergiftung auszuschließen. Darüber hinaus müssen sie einerseits streichfähig sein, andererseits, nach dem Auftragen auf die Lippen aber dort ortsfest verbleiben ohne klebrig zu wirken. Nicht zuletzt müssen diese Produkte mikrobiologisch und thermisch über einen langen Zeitraum stabil sein.

Einen wesentlichen Bestandteil von Lippenpflegeprodukten und anderen Kosmetika stellen seit alters her die Mineralöle und Mineralwachse dar. Diese haben eine angenehme, weiche, cremige Konsistenz, sind geruchslos und geschmacksneutral und sind kostengünstig in großen Mengen von hoher Qualität verfügbar.

Ein Nachteil des Standes der Technik besteht nun in dem Umstand, dass der Einsatz von Mineralölen und -wachsen, nicht ganz unumstritten ist und bei Bewertungen bei einigen Verbrauchermagazinen (z.B. Öko-Test) zu "Abwertungen" in der Benotung des Produktes führen. Begründet wird diese Negativ-Bewertung damit, dass einige Wissenschaftler vermuten, dass Mineralöle und Mineralwachse gesundheitlich nicht unbedenklich sein könnten. Auch wenn, trotz des jahrzehntelangen weltweiten Einsatzes dieser Stoffe keine negativen Wirkungen am Menschen bekannt geworden sind, besteht bei den Verbrauchern der Wunsch, Zubereitungen mit derartigen Inhaltsstoffen zu meiden.

Es war daher die Aufgabe der vorliegenden Erfindung, die Nachteile des Standes der Technik zu beseitigen eine alternative Lipidgrundlage (Ersatzstoff) für Mineralöle und, insbesondere, Mineralwachse zu entwickeln, welche die gleichen sensorischen und formulierungstechnischen Eigenschaften wie diese haben.

Wachse, insbesondere Mineralwachse, dienen in kosmetischen Zubereitungen regelmäßig als Ölbinder, welche die flüssigen Ölkomponenten in einer pastösen oder festen Zubereitung fixieren. Allerdings lassen sich nach dem Stand der Technik nicht alle Öle und Wachse gut miteinander verbinden. Häufig kommt es bei thermischer Belastung oder während einer längeren Lagerungsdauer zu Phasentrennungen von Öl und Wachs. Ferner werden diese Öl-Wachs-Gemische häufig entweder zu weich oder zu fest und spröde, so dass die für das Kosmetikum zu erzielenden rheologischen Eigenschaften häufig verfehlt werden. Dies stellt insbesondere bei Lippenstiften ein zentrales Problem dar, insbesondere bei der Abfüllung der Stiftmasse.

Es war daher die Aufgabe der vorliegenden Erfindung, eine Lipidmischung zu entwickeln, in die sich weitere Öl- und Wachskomponenten problemlos temperatur- und lagerstabil einarbeiten lassen. Insbesondere war es die Aufgabe der vorliegenden Erfindung, eine solche Lipidmischung zu entwickeln, die für Lippenstifte geeignet ist, d.h. die weder zu weich ist, um als Stift formuliert werden zu können noch zu hart und spröde ist, so dass sie sich bequem und in der erforderlichen Menge auf die Lippen auftragen lässt.

Überraschend gelöst werden die Aufgaben durch eine kosmetische Lipidmischung aus
a) Octyldodecanol und
b) hydriertem Rapsöl im Gewichtsverhältnis von 7:1 bis 1:1,
wobei das hydrierte Rapsöl einen Behensäureanteil von 35-60 Gewichts-%, bezogen auf das Gesamtgewicht des hydrierten Rapsöls aufweist

Die erfindungsgemäße Lipidmischung weist eine angenehm cremig, weiche Sensorik auf und Ist geruchs- und geschmacksneutral. Sie ist temperatur- und lagerstabil, d.h. es kommt nicht zu Phasentrennungen.

Zwar kennt der Stand der Technik die WO 99/63031 A1, DE 20309463 U1, WO 2012/131624 A1, WO 2017/008243 A1, DE 102014204204477 A1 sowie den GNPD (Mintel) Datenbankeintrag Nr. 2119122, doch konnten diese Veröffentlichungen nicht den Weg zur vorliegenden Erfindung weisen.

Erfindungsgemäß bevorzugt beträgt das Gewichtsverhältnis von Octyldodecanol zu hydriertem Rapsöl 3:1 bis 1:1 und besonders bevorzugt 2:1.

Es ist ein Behensäureanteil von 42 bis 54 Gewichts-%, bezogen auf das Gesamtgewicht des hydrierten Rapsöls erfindungsgemäß bevorzugt. Der erfindungsgemäß besonders bevorzugte Bereich liegt zwischen 43 und 45 Gewichts-%.

Ferner ist es erfindungsgemäß von Vorteil, wenn das hydrierte Rapsöl als zweithäufigste Fettsäure Stearinsäure enthält. Erfindungsgemäß vorteilhaft liegt der Stearinsäureanteil zwischen 30 und 44 Gewichts-%, bezogen auf das Gesamtgewicht des hydrierten Rapsöls. Der erfindungsgemäß besonders bevorzugte Bereich liegt zwischen 40 und 42 Gewichts-%.

Darüber hinaus enthält das hydrierte Rapsöl erfindungsgemäß vorteilhaft Archidinsäure und/oder Palmitinsäure, wobei ein Gehalt an beiden Säuren erfindungsgemäß bevorzugt ist. Der erfindungsgemäß vorteilhafte Konzentrationsbereich für Archidinsäure liegt dabei zwischen 6 und 10 Gewichts-%, bezogen auf das Gesamtgewicht des hydrierten Rapsöls.

Der erfindungsgemäß vorteilhafte Konzentrationsbereich für Palmitinsäure liegt dabei zwischen 2 und 4 Gewichts-%, bezogen auf das Gesamtgewicht des hydrierten Rapsöls. Erfindungsgemäß ist auch ein Verfahren zur Herstellung dieser Lipidmischung, welches dadurch gekennzeichnet ist, dass das hydrierte Rapsöl im Octyldodecanol unter Rühren aufgeschmolzen wird und anschließend unter Rühren abgekühlt wird.

Erfindungsgemäß bevorzugt beträgt die Temperatur beim Aufschmelzen zwischen 60 und 70° C.

Erfindungsgemäß ist auch eine Lipidmischung, die nach diesem Verfahren hergestellt wird.

Erfindungsgemäß ist nicht zuletzt eine Lippenstiftformulierung enthaltend eine erfindungsgemäße kosmetische Lipidmischung oder eine Lipidmischung, die nach dem erfindungsgemäßen Verfahren hergestellt wurde und dadurch gekennzeichnet ist, dass die Lippenstiftformulierung die Lipidmischung einer Konzentration von 40 bis 60 Gewichts-%, bezogen auf das Gesamtgewicht der Lippenstiftzubereitung enthält. Erfindungsgemäß bevorzugt ist dabei eine Konzentration der Lipidmischung von 45 bis 55 Gewichts-%, bezogen auf das Gesamtgewicht der Lippenstiftformulierung.

Diese erfindungsgemäße Lippenstiftformulierung ist erfindungsgemäß vorteilhaft dadurch gekennzeichnet, dass die Formulierung weitere Wachse gewählt aus der Gruppe der Verbindungen Bienenwachs, Carnaubawachs, Candelillawachs, Sonnenblumenwachs, Reiswachs, hydriertes Rizinusöl enthält. Erfindungsgemäß bevorzugt ist dabei der Einsatz von Bienenwachs und Carnaubawachs.

Es ist vorteilhaft im Sinne der vorliegenden Erfindung, wenn diese weiteren Wachse in einer Gesamtmengen von 0,05 bis 15 Gewichts-%, bezogen auf das Gesamtgewicht der Formulierung enthalten sind.

Die erfindungsgemäß bevorzugten Einsatzkonzentrationen betragen dabei für Bienenwachs von 4 bis Gewichts- 14%, für Carnaubawachs von 0,5 bis 2 Gewichts-%, für Candelillawachs von 0,5 bis 5 Gewichts-%, für Sonnenblumenwachs von 0,5 bis 5 Gewichts-%, für Reiswachs von 0,05 bis 2 Gewichts-% und für hydriertes Rizinusöl von 0,05 bis 5 Gewichts-%, jeweils bezogen auf das Gesamtgewicht er Lippenstiftformulierung.

Ferner ist es für die Lippenstiftformulierung erfindungsgemäß von Vorteil, wenn die Zubereitung Cetylpalmitat, Cetearylalkohol und/oder Sheabutter enthält.

In einem solchen Falle ist es erfindungsgemäß vorteilhaft, wenn die Gesamtmenge an Cetylpalmitat, Cetearylalkohol und Sheabutter in der Formulierung 0,05 bis 15 Gewichts-%, bezogen auf das Gesamtgewicht der Lippenstiftformulierung beträgt.

Die erfindungsgemäß bevorzugten Einsatzkonzentrationen betragen dabei für Cetylpalmitat von 2 bis 10 Gewichts-%, für Cetearylalkohol von 0,5 bis 8 Gewichts-% und für Sheabutter von 0,5 bis 10 Gewichts-%, jeweils bezogen auf das Gesamtgewicht der Lippenstiftformulierung

Erfindungsgemäß vorteilhafte Ausführungsformen der Lippenstiftformulierung sind auch dadurch gekennzeichnet, dass die Formulierung Öle gewählt aus der Gruppe der Verbindungen Riziinusöl, Ethylhexylstearat, Kokosglyceride (INCI Cocoglyceride), Olivenöl und/oder Sonnenblumenöl enthält.

Die erfindungsgemäß bevorzugten Einsatzkonzentrationen betragen dabei für Rizinusöl von 5 bis 20 Gewichts-%, für Ethylhexylstearat von 5 bis 30 Gewichts-%, für Kokosglyceride von 5 bis 30 Gewichts-%, für Olivenöl 1 bis 20 Gewichts-% und für Sonnenblumenöl von 1 bis 10 Gewichts-%, jeweils bezogen auf das Gesamtgewicht der Lippenstiftformulierung

Erfindungsgemäß vorteilhaft kann die erfindungsgemäße Lippenstiftformulierung Pigmente enthalten. Diese sind dann bevorzugt dadurch gekennzeichnet, dass die Formulierung 0,001 bis 3 Gewichts-% Pigmente enthält.

Erfindungsgemäße Pigmente können organischen und anorganischen Ursprungs sein, wie beispielsweise organische vom Azo-Typ, Indigoide, Triphenylmethan-artige, Anthrachinone, und Xanthin Farbstoffe, die als D&C and FD&C blues, browns, greens, oranges, reds, yellows bekannt sind. Anorganische Pigmente bestehen aus unlöslichen Salzen von zertifizierten Farbstoffen, die als Lakes oder Eisenoxide bezeichnet werden. Beispielsweise können Barium lakes, calcium lakes, aluminum lakes, titandioxide, mica and iron oxides Verwendung finden. Als Al-Salze sind z.B Red 3 Aluminum Lake, Red 21 Aluminum Lake, Red 27 Aluminum Lake, Red 28 Aluminum Lake, Red 33 Aluminum Lake, Yellow 5 Aluminum Lake, Yellow 6 Aluminum Lake, Yellow 10 Aluminum Lake, Orange 5 Aluminum Lake, Blue 1 Aluminum Lake und Kombinationen einsetzbar.

Als Eisenoxide oder -oxidhydrate sind z.B. cosmetic yellow oxide C22-8073 (Sunchemical) cosmetic yellow oxide C33-1700 (Sunchemical), cosmetic brown oxide C33-115 (Sunchemical), cosmetic iron oxide red C33-2199 (Sunchemical), cosmetic russet oxide C33-8075 (Sunchemical), cosmetic iron oxide black C33-5000 (Sunchemical), als Titandioxide z.B. Kronos 1171 (Kronos), C47-051 Cosmetic White (Sunchemical) bekannt und gegebenfalls vorteilhaft. Ebenso ist es vorteilhaft, wenn diese Zubereitungen einen Gehalt an anorganischen Pigmenten gewählt aus der Gruppe der Metalloxide und/oder anderen in Wasser schwerlöslichen oder unlösliche Metallverbindungen, bevorzugt Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄) aufweisen.

Die anorganischen Pigmente können vorteilhaft im Sinne der vorliegenden Erfindung auch in Form kommerziell erhältlicher öliger oder wässriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß besonders bevorzugt ist es, wenn die Formulierung Silica, Mica oder Titandioxid als Pigmente enthält.

Es ist erfindungsgemäß von Vorteil, wenn die Lippenstiftformulierung hydrophile Bestandteile in einer Gesamtmenge von 0,001 bis 1 Gewichts-%, bezogen auf das Gesamtgewicht der Formulierung enthält.

In einem solchen Falle ist es dann erfindungsgemäß bevorzugt, wenn die Formulierung als hydrophile Bestandteile Wasser, Panthenol und/oder Glycerin enthält.

Die erfindungsgemäße Lippenstiftformulierung ist erfindungsgemäß vorteilhaft auch dadurch gekennzeichnet, dass die Formulierung Polyglyceryl-3 Diisostearat enthält.
In einem solchen Falle beträgt die erfindungsgemäß vorteilhafte Einsatzkonzentration von Polyglyceryl-3 Diisostearat von 0,5 bis 5 Gewichts-%, bezogen auf das Gesamtgewicht der Formulierung.

Erfindungsgemäß vorteilhafte Ausführungsformen der erfindungsgemäßen Lippenstiftformulierung sind nicht zuletzt dadurch gekennzeichnet, dass die Formulierung Aromen (Aromastoffe) und/oder Parfümstoffe enthält.

Es ist dann erfindungsgemäß von Vorteil, wenn die Aromastoffe aus der Gruppe der Ester gewählt werden und bevorzugt aus der Gruppe der Acetate und Butyrate. Dabei ist es erfindungsgemäß bevorzugt, wenn als Aromastoff Isopentylacetat eingesetzt wird.

Erfindungsgemäß vorteilhafte Ausführungsformen sind auch dadurch gekennzeichnet, dass die die Lippenstiftformulierung Antioxidantien enthält.

Als erfindungsgemäß bevorzugte Antioxidantien werden dabei BHT (Butylhydroxytoluol), Tocopherol und Tocopherolacetat angesehen.

Die erfindungsgemäße Lippenstiftformulierung kann erfindungsgemäß weitere Bestandteile enthalten. So ist es erfindungsgemäß vorteilhaft, wenn die erfindungsgemäße Zubereitung als weitere Bestandteile eine oder mehrere Verbindungen gewählt aus der Gruppe der UV-Lichtschutzfilter (z.B. Ethylhexylmethoxycinnamat und/oder Butylmethoxydibenzoylmethan), Vitamin-A-Palmitat, Vitamin-E-Acetat, Allantoin, Panthenol, α-Bisabolol, Lecithin, Ceramide , Collagene, Ubiquinone, sowie pflegende Öle auf pflanzlicher Basis enthält

Es ist erfindungsgemäß bevorzugt, wenn die erfindungsgemäße Lippenstiftformulierung frei ist von Mineralölen.

Erfindungsgemäß vorteilhaft ist es ferner, wenn die Zubereitung frei ist von Polyethylenglycolen und deren Derivaten.

Erfindungsgemäß vorteilhafte Ausführungsformen zeichnen sich darüber hinaus dadurch aus, dass die Zubereitung frei ist von Parabenen.

Nicht zuletzt sind die erfindungsgemäßen Zubereitungen erfindungsgemäß vorteilhaft frei ist von Silikonölen oder -wachsen.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### a) Beispiele Lipidmischung

Es wurde das hydrierte Rapsöl bei 70 °C in das Octyldodecanol eingerührt und unter Rühren auf 25 °C abgekühlt.

### Bespiel 1 - Lipidmischung

| | | (%) |
|---|---|---|
| A | Octyldodecanol | 70 |
| B | hydrogenated Rapeseed Oil | 30 |
| | | 100 |

### Bespiel 2 - Lipidmischung

| | | (%) |
|---|---|---|
| A | Octyldodecanol | 85 |
| B | hydrogenated Rapeseed Oil | 15 |
| | | 100 |

### Bespiel 3 - Lipidmischung

| | | (%) |
|---|---|---|
| A | Octyldodecanol | 54 |
| B | hydrogenated Rapeseed Oil | 46 |
| | | 100 |

### b) Beispiele für die Lippenstiftformulierung

Es wurde die Lipidmischung und dessen Mischungsverhältnis aus Beispiel 1 nun in die folgende Stiftformulierung eingebaut.

### 1) fester Lippenpflegestift

| | | (%) |
|---|---|---|
| A | Octyldodecanol | 35 |
| | Hydrogenated Rapeseed Oil | 15 |
| | | |
| B | Ethylhexyl Stearate | 15 |
| | Ricinus Communis Seed Oil | 10 |
| | | |
| C | Butyrospermum Parkii Butter | 2 |
| | Cera Alba | 11,5 |
| | Copernicia Cerifera Cera | 0,5 |
| | Cetearyl Alcohol | 3 |
| | Cetyl Palmitate | 5 |
| | | |
| D | Polyglyceryl-3 Diisostearate | 3 |
| | Parfuem / Aroma | add to 100 |
| | Pigment | add to 100 |
| | Persea Gratissima Oil | add to 100 |
| | Simmondsia Chinensis seed oil | add to 100 |
| | Neohesperidin Dihydrochalcone | add to 100 |
| | UV filter | add to 100 |
| | BHT | add to 100 |
| | | 100 |

Zuerst wird Phase A auf circa 60 - 70°C erhitzt, um das hydrierte Rapsöl in Octyldodecanol zu schmelzen.

In einem separaten Becherglas wird nun Phase B auf 80-90°C erhitzt, bei erreichter Temperatur wird Phase C hinzugegeben und geschmolzen. Liegen diese Phasen und alle hochschmelzenden Wachse klar gelöst vor, kann diese auf circa 60 -65°C heruntergekühlt werden. Schließlich wird Phase A bei erreichter Temperatur hinzugegeben, nun können auch die weitere Phase D, wie zum Beispiel der Emulgator mit dem Aroma, beigemengt werden.

Vergossen werden kann die Masse bei nun circa 60°C in eine Form. Jetzt sollte sie auskühlen und erst im vollständig erstarrtem Zustand entformt werden.

### 2) kosmetischer Stift

| | | (%) |
|---|---|---|
| A | Octyldodecanol | 47 |
| | Hydrogenated Rapeseed Oil | 10 |
| | | |
| B | Ethylhexyl Stearate | 7 |
| | Ricinus Communis Seed Oil | 10 |
| | | |
| C | Butyrospermum Parkii Butter | 5 |
| | Candelilla Cera | 0,5 |
| | Cera Alba | 14 |
| | Copernicia Cerifera Cera | 0,5 |
| | Cetearyl Alcohol | 3 |
| | Helianthus Annuus Seed Cera | 0,5 |
| | | |
| D | Polyglyceryl-3 Diisostearate | 2,5 |
| | Parfuem / Aroma | add to 100 |
| | Pigment | add to 100 |
| | Panthenol | add to 100 |
| | UV filter | add to 100 |
| | BHT | add to 100 |
| | | 100 |

Die Herstellung erfolgt analog der Rezeptur fester Lippenpflegestift.

## Patentansprüche

1. Kosmetische Lipidmischung aus
a) Octyldodecanol und
b) hydriertem Rapsöl im Gewichtsverhältnis von 7:1 bis 1:1, wobei das hydrierte Rapsöl einen Behensäureanteil von 35-60 Gewichts-%, bezogen auf das Gesamtgewicht des hydrierten Rapsöls aufweist.

2. Kosmetische Lipidmischung nach Anspruch 1, **dadurch gekennzeichnet, dass** das hydrierte Rapsöl als zweithäufigste Fettsäure Stearinsäure enthält.

3. Verfahren zur Herstellung einer Lipidmischung nach einem der Ansprüche 1 und 2
**dadurch gekennzeichnet, dass** das hydrierte Rapsöl im Octyldodecanol unter Rühren aufgeschmolzen wird und anschließend unter Rühren abgekühlt wird.

4. Lipidmischung hergestellt nach einem Verfahren nach Anspruch 3.

5. Lippenstiftformulierung enthaltend eine kosmetische Lipidmischung nach einem der Ansprüche 1, 2 oder 4, oder hergestellt nach einem Verfahren nach Anspruch 3,
**dadurch gekennzeichnet dass** die Lippenstiftformulierung die Lipidmischung einer
Konzentration von 40 bis 60 Gewichts-%, bezogen auf das Gesamtgewicht der
Lippenstiftzubereitung enthält.

6. Lippenstiftformulierung nach Anspruch 5, **dadurch gekennzeichnet, dass** die
Formulierung weitere Wachse gewählt aus der Gruppe der Verbindungen
Bienenwachs, Carnaubawachs, Candelillawachs, Sonnenblumenwachs, Reiswachs,
hydriertes Rizinusöl enthält.

7. Lippenstiftformulierung nach Anspruch 6, **dadurch gekennzeichnet, dass** die weiteren Wachse in einer Gesamtmenge von 0,05 bis 15 Gewichts-%, bezogen auf das
Gesamtgewicht der Formulierung enthalten sind.

8. Lippenstiftformulierung nach einem der Ansprüche 5 bis 7, **dadurch gekennzeichnet,**
**dass** die Zubereitung Cetylpalmitat, Cetearylalkohol und/oder Sheabutter enthält.

9. Lippenstiftformulierung nach Anspruch 8, **dadurch gekennzeichnet, dass** die
Gesamtmenge an Cetylpalmitat, Cetearylalkohol und Sheabutter in der Formulierung 0,05 bis 15 Gewichts-%, bezogen auf das Gesamtgewicht der Formulierung beträgt.

10. Lippenstiftformulierung nach einem der Ansprüche 5 bis 9, **dadurch gekennzeichnet,**
**dass** die Formulierung Öle gewählt aus der Gruppe der Verbindungen Rizinusöl,
Ethylhexylstearat, Kokosglyceride (INCI Cocoglyceride), Olivenöl und/oder
Sonnenblumenöl enthält.

11. Lippenstiftformulierung nach einem der Ansprüche 5 bis 10, **dadurch gekennzeichnet,**
**dass** die Formulierung 0,001 bis 3 Gewichts-% Pigmente enthält.

12. Lippenstiftformulierung nach einem der Ansprüche 5 bis 11, **dadurch gekennzeichnet,**
**dass** die Formulierung Silica, Mica oder Titandioxid als Pigmente enthält.

13. Lippenstiftformulierung nach einem der Ansprüche 5 bis 12, **dadurch gekennzeichnet, dass** die Formulierung hydrophile Bestandteile in einer Gesamtmenge von 0,001 bis 1 Gewichts-%, bezogen auf das Gesamtgewicht der Formulierung enthält.

14. Lippenstiftformulierung nach einem der Ansprüche 5 bis 13, **dadurch gekennzeichnet, dass** die Formulierung als hydrophile Bestandteile Wasser, Panthenol und/oder Glycerin enthält.

15. Lippenstiftformulierung nach einem der Ansprüche 5 bis 14, **dadurch gekennzeichnet, dass** die Formulierung Polyglyceryl-3 Diisostearat enthält.

16. Lippenstiftformulierung nach einem der Ansprüche 5 bis 15, **dadurch gekennzeichnet, dass** die Formulierung Polyglyceryl-3 Diisostearat in einer Konzentration von 0,5 bis 5 Gewichts-%, bezogen auf das Gesamtgewicht der Formulierung, enthält.

17. Lippenstiftformulierung nach einem der Ansprüche 5 bis 16, **dadurch gekennzeichnet, dass** die Formulierung Aromen (Aromastoffe) und/oder Parfümstoffe enthält.

18. Lippenstiftformulierung nach einem der Ansprüche 5 bis 17, **dadurch gekennzeichnet, dass** die Formulierung frei ist von Mineralölen.

## Claims

1. Cosmetic lipid mixture consisting of
a) octyldodecanol and
b) hydrogenated rapeseed oil in a weight ratio of 7:1 to 1:1, wherein the hydrogenated rapeseed oil has a behenic acid content of 35-60% by weight based on the total weight of the hydrogenated rapeseed oil.

2. Cosmetic lipid mixture according to Claim 1, **characterized in that** the fatty acid with the second-highest content in the hydrogenated rapeseed oil is stearic acid.

3. Process for the production of a lipid mixture according to either of Claims 1 and 2, **characterized in that** the hydrogenated rapeseed oil is melted in the octyldodecanol with stirring and then cooled with stirring.

4. Lipid mixture produced according to a process according to Claim 3.

5. Lipstick formulation containing a cosmetic lipid mixture according to any of Claims 1, 2 or 4 or produced according to a process according to Claim 3, **characterized in that** the lipstick formulation contains the lipid mixture in a concentration of 40 to 60% by weight based on the total weight of the lipstick preparation.

6. Lipstick formulation according to Claim 5, **characterized in that** the formulation contains further waxes selected from the group of compounds consisting of beeswax, carnauba wax, candelilla wax, sunflower wax, rice wax, hydrogenated castor oil.

7. Lipstick formulation according to Claim 6, **characterized in that** the further waxes are present in a total amount of 0.05 to 15% by weight based on the total weight of the formulation.

8. Lipstick formulation according to any of Claims 5 to 7, **characterized in that** the preparation contains cetyl palmitate, cetearyl alcohol, and/or shea butter.

9. Lipstick formulation according to Claim 8, **characterized in that** the total amount of cetyl palmitate, cetearyl alcohol, and shea butter in the formulation is 0.05 to 15% by weight based on the total weight of the formulation.

10. Lipstick formulation according to any of Claims 5 to 9, **characterized in that** the formulation contains oils selected from the group of compounds consisting of castor oil, ethylhexyl stearate, coconut glycerides (INCI Cocoglycerides), olive oil, and/or sunflower oil.

11. Lipstick formulation according to any of Claims 5 to 10, **characterized in that** the formulation contains 0.001 to 3% by weight of pigments.

12. Lipstick formulation according to any of Claims 5 to 11, **characterized in that** the formulation contains silica, mica or titanium dioxide as pigments.

13. Lipstick formulation according to any of Claims 5 to 12, **characterized in that** the formulation contains hydrophilic constituents in a total amount of 0.001 to 1% by weight based on the total weight of the formulation.

14. Lipstick formulation according to any of Claims 5 to 13, **characterized in that** the formulation contains water, panthenol, and/or glycerol as hydrophilic constituents.

15. Lipstick formulation according to any of Claims 5 to 14, **characterized in that** the formulation contains polyglyceryl-3 diisostearate.

16. Lipstick formulation according to any of Claims 5 to 15, **characterized in that** the formulation contains polyglyceryl-3 diisostearate in a concentration from 0.5 to 5% by weight based on the total weight of the formulation.

17. Lipstick formulation according to any of Claims 5 to 16, **characterized in that** the formulation contains flavorings (flavoring agents) and/or perfumes.

18. Lipstick formulation according to any of Claims 5 to 17, **characterized in that** the formulation is free of mineral oils.

## Revendications

1. Mélange de lipides cosmétique composé
a) d'octyldodécanol et
b) d'huile de colza hydrogénée en un rapport en poids de 7 : 1 à 1 : 1, l'huile de colza hydrogénée présentant une proportion en acide béhénique de 35 à 60 % en poids, par rapport au poids total de l'huile de colza hydrogénée.

2. Mélange de lipides cosmétique selon la revendication 1, **caractérisé en ce que** l'huile de colza hydrogénée contient de l'acide stéarique en tant que deuxième acide gras le plus abondant.

3. Procédé pour la préparation d'un mélange de lipides selon l'une quelconque des revendications 1 et 2 **caractérisé en ce que** l'huile de colza hydrogénée est fondue dans l'octyldodécanol sous agitation et ensuite on refroidit sous agitation.

4. Mélange de lipides préparé par un procédé selon la revendication 3.

5. Formulation de bâton à lèvres contenant un mélange de lipides cosmétique selon l'une quelconque des revendications 1, 2 et 4, ou préparé par un procédé selon la revendication 3, **caractérisée en ce que** la formulation de bâton à lèvres contient le mélange de lipides en une concentration de 40 à 60 % en poids, par rapport au poids total de la préparation de bâton à lèvres.

6. Formulation de bâton à lèvres selon la revendication 5, **caractérisée en ce que** la formulation contient des cires supplémentaires choisies dans le groupe des composés cire d'abeilles, cire de carnauba, cire de candelilla, cire de tournesol, cire de riz, huile de ricin hydrogénée.

7. Formulation de bâton à lèvres selon la revendication 6, **caractérisée en ce que** les cires supplémentaires sont contenues en une quantité totale de 0,05 à 15 % en poids, par rapport au poids total de la formulation.

8. Formulation de bâton à lèvres selon l'une quelconque des revendications 5 à 7, **caractérisée en ce que** la préparation contient du palmitate de cétyle, de l'alcool cétéarylique et/ou du beurre de karité.

9. Formulation de bâton à lèvres selon la revendication 8, **caractérisée en ce que** la quantité totale de palmitate de cétyle, d'alcool cétéarylique et de beurre de karité dans la formulation est de 0,05 à 15 % en poids, par rapport au poids total de la formulation.

10. Formulation de bâton à lèvres selon l'une quelconque des revendications 5 à 9, **caractérisée en ce que** la formulation contient des huiles choisies dans le groupe des composés huile de ricin, stéarate d'éthylhexyle, glycérides de coco (INCI Cocoglyceride), huile d'olive et/ou huile de tournesol.

11. Formulation de bâton à lèvres selon l'une quelconque des revendications 5 à 10, **caractérisée en ce que** la formulation contient 0,001 à 3 % en poids de pigments.

12. Formulation de bâton à lèvres selon l'une quelconque des revendications 5 à 11, **caractérisée en ce que** la formulation contient de la silice, du mica ou du dioxyde de titane en tant que pigments.

13. Formulation de bâton à lèvres selon l'une quelconque des revendications 5 à 12, **caractérisée en ce que** la formulation contient des ingrédients hydrophiles en une quantité totale de 0,001 à 1 % en poids, par rapport au poids total de la formulation.

14. Formulation de bâton à lèvres selon l'une quelconque des revendications 5 à 13, **caractérisée en ce que** la formulation contient en tant qu'ingrédients hydrophiles de l'eau, du panthénol et/ou de la glycérine.

15. Formulation de bâton à lèvres selon l'une quelconque des revendications 5 à 14, **caractérisée en ce que** la formulation contient du diisostéarate de polyglycéryle-3.

16. Formulation de bâton à lèvres selon l'une quelconque des revendications 5 à 15, **caractérisée en ce que** la formulation contient du diisostéarate de polyglycéryle-3 en une concentration de 0,5 à 5 % en poids, par rapport au poids total de la formulation.

17. Formulation de bâton à lèvres selon l'une quelconque des revendications 5 à 16, **caractérisée en ce que** la formulation contient des arômes (substances aromatiques) et/ou des matières parfumées.

18. Formulation de bâton à lèvres selon l'une quelconque des revendications 5 à 7, **caractérisée en ce que** la formulation est exempte d'huiles minérales.
